# FASCICULE DE BREVET EUROPEEN

(11) **EP 1 043 285 B1**
(45) Date de publication et mention de la délivrance du brevet: **01.10.2003**
(21) Numéro de dépôt: 00400936.1
(22) Date de dépôt: 05.04.2000
(51) Int. Cl.: C04B 24/02, C04B 24/10, C07H 3/00

(54) **Adjuvants pour liants minéraux, à base d'un produit de déshydratation interne d'un sucre hydrogéné, liants minéraux adjuvantés et leur procédé de préparation**
Zusatzstoff für mineralisches Bindemittel auf Basis eines Produkts der internen Entwässerung von hydrogeniertem Zucker, diesen Zusatzstoff enthaltendes Mineralbindemittel und Verfahren zur Herstellung
Additive for mineral binder based on the product of internal dehydration of a hydrogenated sugar, mineral binders containing said additive and method for preparing them

(30) Priorité: 08.04.1999 FR 9904393
(43) Date de publication de la demande: 11.10.2000
(73) Titulaire: Roquette Frères, 62136 Lestrem (FR)
(72) Inventeur: Mentink, Léon, 59800 Lille (FR); Graux, Jean-Pierre, 62190 Lillers (FR)
(74) Mandataire: Boulinguiez, Didier

(56) Documents cités:
- EP-A- 0 650 941
- DE-A- 2 149 601
- GB-A- 1 085 594
- DATABASE WPI Section Ch, Week 198434 Derwent Publications Ltd., London, GB; Class E17, AN 1984-209683 XP002126264 & JP 59 121143 A (SUMITOMO CHEM CO LTD), 13 juillet 1984 (1984-07-13)
- DATABASE WPI Section Ch, Week 198429 Derwent Publications Ltd., London, GB; Class A96, AN 1984-180130 XP002126265 & JP 59 101410 A (SANKIN KOGYO KK), 12 juin 1984 (1984-06-12)
- DATABASE WPI Section Ch, Week 197426 Derwent Publications Ltd., London, GB; Class E13, AN 1974-47997V XP002126266 & JP 49 021409 B (NIPPON STEEL CORP), 31 mai 1974 (1974-05-31)

## Description

La présente invention a pour objet des adjuvants pour les liants minéraux, à base d'une composition contenant au moins un produit de déshydratation interne d'un sucre hydrogéné. Elle a également pour objet les liants minéraux adjuvantés ainsi que l'utilisation desdits adjuvants ou desdites compositions pour la préparation de liants minéraux.

Par " liant minéral ", on entend, au sens de la présence invention, tout liant hydraulique, notamment toute poudre minérale, apte à former avec l'eau une pâte faisant prise et durcissant progressivement, même à l'abri de l'air. Classiquement, à température ambiante, un liant minéral commence à former avec l'eau une telle pâte dans un délai d'environ quelques minutes à moins de 48 heures, généralement entre environ 30 minutes et 24 heures. Cette définition s'applique, entre autres, aux ciments, aux chaux hydrauliques naturelles ou artificielles, mais aussi aux mélanges, pâteux ou durcis, tels que mortiers, coulis, enduits et bétons, à base de ciment broyé et/ou de chaux, d'eau et/ou de granulats (sables, graviers, cailloux,...), et enfin aux matières premières entrant dans la fabrication des ciments tels que pouzzolanes, clinkers, laitiers, fillers calcaires et fumées de silice. Par " liant minéral ", on entend aussi tout liant non hydraulique à base de sulfate de calcium, de gypse et/ou de chaux.

En fonction de leurs utilisations finales et conditions d'usage, il est parfois nécessaire d'ajouter aux liants minéraux des adjuvants tels que les agents de mouture ou auxiliaires de broyage, les accélérateurs de prise et/ou de durcissement, les retardateurs de prise, les plastifiants, les réducteurs d'eau-plastifiants, les super plastifiants et les épaississants. Ces adjuvants permettent par exemple de modifier la fluidité, la pompabilité, la maniabilité, la prise, le durcissement, la résistance, la durabilité et/ou certaines autres propriétés du liant minéral.

De nombreux sucres et dérivés sont déjà utilisés dans la préparation d'adjuvants pour liants minéraux, dont :
- les mélasses, qui sont des produits peu coûteux, utilisés comme plastifiants ;
- les sucres, qui sont de bons plastifiants réducteurs d'eau mais fortement retardateurs de prise ;
- les sucres oxydés, très bons plastifiants réducteurs d'eau, retardateurs de prise, et qui permettent également d'améliorer la résistance à 28 jours comme décrit dans les brevets FR N° 2 387 194 et GB N° 1 508 761 ;
- les sucres hydrogénés, qui sont des plastifiants-réducteurs d'eau comme décrit dans le brevet FR 2.726.550, mais qui sont cependant moins retardateurs que les sucres oxydés tout en améliorant la résistance à 28 jours, comme il résulte du brevet américain US N° 4 073 658 ;
- les sirops de sucre hydrogénés ou oxydés qui sont également décrits comme agents de mouture, selon le brevet EP N° 0 696 557 ;
- les esters d'acides gras supérieurs et de polyols tels que le trioléate de sorbitan qui sont des agents de contrôle de la capacité d'absorption d'eau et agents d'amélioration de l'étanchéité à l'eau et de l'adhérence de compositions de ciment à l'état durci comme décrit dans le brevet japonais JP n° 59121143. Ces esters d'acides gras supérieurs apparaissent en outre comme étant des retardateurs de prise et/ou de durcissement.

Actuellement, pour la préparation des ciments, la tendance est à utiliser des produits moins chers que le clinker et/ou à réduire l'emploi de ce dernier. Cependant, le clinker confère une bonne résistance à 28 jours. Il existe donc un besoin en un adjuvant permettant de corriger la résistance à 28 jours de ciments ne contenant pas ou contenant peu de clinker.

Par ailleurs, en ce qui concerne les mortiers, coulis et bétons, l'industrie est à la recherche de nouveaux accélérateurs de prise et/ou de durcissement.

Les accélérateurs de prise et/ou de durcissement classiquement utilisés jusqu'à ce jour sont, d'une part des produits chlorés comme par exemple le chlorure de calcium, et d'autre part des produits non chlorés, fortement acides ou basiques, comme par exemple respectivement les acides formique ou sulfurique, leurs sels, la chaux, la soude ou les produits en générant (métasilicates ou aluminates de sodium).

De tels accélérateurs sont couramment mis en oeuvre, notamment lors de la préparation de liants minéraux destinés à être utilisés à basses températures, i.e à des températures inférieures à 15°C environ, ou lors de la réalisation, en usine, d'articles préfabriqués.

Cependant, les produits chlorés ont pour inconvénients majeurs d'être corrosifs vis à vis des enceintes de préparation des liants et des armatures métalliques utilisées au sein des bétons employés dans le génie civil ou la construction de bâtiments et d'être une source de chlore, produit connu pour être nocif à l'environnement.

De leur côté, les produits fortement acides ou basiques présentent les inconvénients d'être corrosifs vis à vis des métaux et agressifs vis à vis de la peau et des yeux et de ne pas toujours permettre d'obtenir des résistances suffisamment améliorées au jeune âge et/ou à 28 jours.

Des produits neutres et non chlorés sont parfois utilisés en tant qu'accélérateurs de prise et/ou de durcissement comme, par exemple, le carbonate de lithium. Cependant, ce composé présente l'inconvénient d'être cher par rapport aux produits précités.

En outre, il a tendance à réduire significativement la plasticité des liants minéraux et d'être peu efficace aux basses températures.

Il existe donc un besoin de pouvoir disposer d'un adjuvant pour liants minéraux qui, simultanément
- soit écologique, inoffensif lors de son usage et non corrosif vis à vis des métaux,
- soit accélérateur de prise et/ou de durcissement et permette par conséquent d'obtenir des résistances au jeune âge améliorées et suffisantes pour rendre possible des décoffrages rapides et ce, tant aux températures ordinaires qu'aux basses températures,
- confère une plasticité correcte, si possible améliorée, aux liants minéraux lors de leur mise en oeuvre, et
- confère ensuite à ces liants des propriétés mécaniques à 28 jours, correctes et si possible améliorées.

Dans le cadre de la présente invention, on entend par plasticité du liant minéral la capacité d'obtenir un état rhéologique dans lequel le liant minéral est manipulable, coulable ou pompable. La mesure de la plasticité est effectuée selon la méthode normalisée CEN 196-01 par laquelle on mesure, en mm, l'étalement d'un volume donné de liant minéral sur une table à chocs.

Le début et la fin de prise sont mesurés à l'aide d'un prisomètre automatique de marque " ACMEL ".

La résistance mécanique au jeune âge est mesurée sur éprouvettes de liant minéral selon la norme CEN 196-01 précitée et ce, 17 ou 24 heures après la fabrication des éprouvettes. Cette résistance mécanique au jeune âge doit, en général, être supérieure à 5 Mpa pour permettre un décoffrage. Par ailleurs, la résistance dans le temps des liants minéraux (par exemple à 3 ou 28 jours) est également mesurée selon la norme précitée CEN 196-01.

La société déposante a eu le mérite de trouver, après de nombreuses recherches, qu'un adjuvant répondant aux exigences précitées de la technique actuelle, pouvait consister en une composition particulière contenant un dérivé de sucre sélectionné.

De manière plus précise la présente invention a pour objet un nouvel adjuvant pour liants minéraux caractérisé en ce qu'il comprend une composition contenant au moins un produit de déshydratation interne d'un sucre hydrogéné, à l'exclusion du produit résultant de l'estérification par des chaînes grasses dudit produit de déshydratation interne. De façon surprenante et inattendue de tels produits de déshydratation se comportent de manière totalement différente des sucres hydrogénés dont ils dérivent et qui sont connus comme étant des agents retardateurs comme rappelé ci-avant.

Par " produit de déshydratation interne " on entend tout produit résultant, d'une manière quelconque, en une ou plusieurs étapes, de l'enlèvement d'une ou de plusieurs molécules d'eau au niveau de la structure interne originelle d'un sucre hydrogéné ainsi que tout composé contenant, du fait notamment de phénomènes de (poly)condensation éventuels, un tel produit. On entend également tout composé résultant de la modification chimique et par exemple de l'éthérification d'un tel produit ou de son estérification par une chaîne non grasse, comme mentionné ci-avant. De tels composés peuvent consister notamment en des dérivés acétylés, éthylés, méthylés, propylés, butylés, éthylénés ou propylénés de l'isosorbide ou de l'isomannide. Il peut s'agir en particulier de di-méthyl isosorbide.

De préférence, le sucre hydrogéné est un mondsaccharide hydrogéné, notamment choisi parmi les hexitols, les pentitols, les tétritols et leurs mélanges. Ils peut s'agir également d'un di-, oligo- ou polysaccharide hydrogéné ou d'un mélange quelconque de ces produits.

Selon une variante particulièrement avantageuse, le produit de déshydratation est issu de la déshydratation interne plus ou moins poussée, d'un hexitol comme par exemple le sorbitol, le mannitol ou le galactitol et consiste notamment en un hexitol déshydraté choisi parmi 1' isosorbide, l'isomannide, le sorbitan, le mannitan et les mélanges quelconques d'au moins deux quelconques de ces produits.

Selon une autre variante, la composition contenue dans l'adjuvant selon l'invention présente une teneur en produit(s) de déshydratation d'au moins 5 %, de préférence d'au moins 10 %, et plus préférentiellement d'au moins 40 %, ces pourcentages étant exprimés en poids sec total de produit(s) de déshydratation par rapport au poids sec de ladite composition.

La Société Demanderesse a trouvé, comme il sera exemplifié ci-après, que des compositions présentant une teneur totale en isosorbide et sorbitan d'au moins 15 %, de préférence d'au moins 55 %, et plus préférentiellement encore d'au moins 70 %, étaient particulièrement d'intérêt comme agents accélérateurs de prise et/ou de durcissement de liants minéraux ou comme agents améliorateurs des propriétés mécaniques de ces liants.

Et il est remarquable de souligner que de telles propriétés insoupçonnables peuvent être obtenues :
- pour des taux d'introduction en ces compositions au sein des liants minéraux, généralement plus faibles que les accélérateurs de prise et/ou de durcissement classiques précités, et
- aussi bien à des températures ordinaires, i.e égales ou supérieures à 15°C environ, qu'aux basses températures,

La présente invention a également pour objet un liant minéral caractérisé en ce qu'il contient de 0,001 à 5 % environ, de préférence de 0,01 à 2 %, d'un adjuvant tel que décrit ci-avant, ces pourcentages étant exprimés en poids sec dudit adjuvant par rapport au poids sec total de matière(s) première(s) pour ciment, de ciment et/ou de chaux contenu dans ledit liant minéral.

Des taux d'introduction en adjuvant de l'ordre de 0,1 à 1 % peuvent généralement convenir à l'ensemble des liants minéraux et à l'ensemble des conditions, y compris de température, de leur mise en place et durcissement.

L'adjuvant selon l'invention peut être, ou non, constitué entièrement de la composition à base de produit(s) de déshydratation décrite ci-avant.

Dans ce cas, ladite composition peut se présencer sous forme liquide, pâteuse ou solide.

Les formes non solides peuvent présenter une gamme très large de matières sèches (MS), par exemple des MS se situant entre environ 30 et 85 %.

La proportion de produit(s) de déshydratation tels que susdécrits par rapport à la MS totale de cette composition, peut varier également très fortement comme déjà explicité.

Ces MS et proportions dépendent notamment du procédé d'obtention de ladite composition. A titre purement exemplatif et aucunement limitatif, cette composition peut consister :
- en un milieu réactionnel non purifié, ci-après désigné " PRODUIT A ", issu de la déshydratation classique du sorbitol sous l'effet d'un acide et de la température, ce milieu présentant une MS de 50 % environ et une teneur totale en isosorbide + sorbitan de 75 % environ (sec/sec),
- en une poudre d'isosorbide cristallisée de très haute pureté (pureté = 99 %), ci-après désigné " PRODUIT B ", issu de la distillation directe du PRODUIT A,
- en le résidu de distillation obtenu conjointement au PRODUIT 3, ci-après désigné " PRODUIT C " présentant une MS de 58 % environ et une teneur totale en isosorbide + sorbitan de 19 % environ (sec/sec),
- en un PRODUIT A enrichi en isosorbide et sorbitan, ci-après désigné " PRODUIT D ", présentant une MS de 79 % environ et une teneur totale en isosorbide + sorbitan de 89 % environ (sec/sec),
- en un milieu enrichi issu d'une double distillation du PRODUIT D, ci-après désigné " PRODUIT E ", présentant une MS de 83 % environ et une teneur totale en isosorbide + sorbitan de 99 % environ,
- en le résidu de ladite double distillation tel qu'obtenu conjointement au PRODUIT E, ci-après désigné " PRODUIT F ", ce résidu présentant une MS de 42 % environ et une teneur totale en isosorbide + sorbitan de 89 % environ,
- en du sorbitan pulvérulent, d'une pureté de 95 % environ ci-après désigné " PRODUIT G ", et
- en de l'isomannide pulvérulent, d'une pureté de 95 % environ ci-après désigné " PRODUIT H ".

De telles compositions, utilisables conformément à l'invention, peuvent présenter des pH se situant dans une large gamme et en particulier entre 2,5 et 6,5 environ.

L'adjuvant selon l'invention peut également contenir, outre ladite composition, un ou plusieurs adjuvants utilisés traditionnellement pour la préparation de liants minéraux, y compris au moins un accélérateur de prise et/ou de durcissement classique tel que ceux précités.

La Demanderesse a notamment observé qu'il pouvait être avantageux d'associer un produit de déshydratation interne d'un sucre hydrogéné tel que l'isosorbide à de la chaux ou du sulfate de sodium.

L'adjuvant ainsi obtenu peut également se présenter sous forme liquide, pâteuse ou solide.

D'une manière générale, l'adjuvant selon l'invention, constitué ou non de la seule composition à base de produit(s) de déshydratation interne de sucre(s) hydrogéné(s), est tout à fait approprié pour être utilisé comme adjuvant pour ciment et ce, avant, pendant et /ou après broyage, ou adjuvant pour les chaux hydrauliques. Il est également tout à fait approprié pour être utilisé comme adjuvant pour bétons, coulis et mortiers, qu'ils soient liquides ou solides.

L'adjuvant selon l'invention peut être introduit au sein des liants minéraux selon une multitude de variantes. Il peut l'être en totalité lors d'une étape particulière de fabrication, de stockage, d'adjuvantation, de transport ou de mise en place du liant minéral ou de manière fractionnée lors de plusieurs de ces étapes particulières. Il peut, par exemple, être utilisé, en tout ou partie, lors de la fabrication de liants minéraux pulvérulents, y compris au niveau même des cimenteries, avant, pendant et/ou après broyage ou lors de la préparation de mortiers ou bétons secs et/ou prêts à l'emploi. Il peut également être utilisé, en tout ou partie, lors du transport de liants minéraux pâteux ou liquides, ou de leur fabrication en usine ou sur chantier, et par exemple dans l'eau de gâchage et/ou les granulats nécessaires à la préparation, notamment dans des enceintes appelées communément " centrales à béton ", de mortiers.

En suite de quoi la présente invention a également pour objets, respectivement :
- l'utilisation d'un tel adjuvant pour la préparation d'un liant minéral, et
- l'utilisation d'une composition contenant au moins un produit de déshydratation interne d' un sucre hydrogéné, en particulier d'un hexitol déshydraté, à l'exclusion du produit résultant de l'estérification par des chaînes grasses dudit produit de déshydratation interne d'un sucre hydrogéné, pour la préparation d'un tel adjuvant ou d'un liant minéral.

Le concept général de la présente invention repose également sur l'utilisation, surprenante et inattendu, d'une telle composition, seule ou au sein d'un adjuvant plus complexe, comme :
- agent accélérateur de prise et/ou de durcissement d'un liant minéral, et/ou
- agent améliorateur des propriétés mécaniques d'un liant minéral à l'état durci.

Et il est remarquable de noter, comme déjà souligné, que de telles fonctionnalités avantageuses s'expriment aussi bien aux températures ordinaires qu'aux basses températures.

La présente invention va être décrite de façon encore plus détaillée à l'aide des exemples qui suivent et qui ne sont aucunement limitatifs.

Dans tous les exemples qui suivent, les mesures de l'étalement (en mm) et de résistances mécaniques (en MPa) à 17 heures, 24 heures, 3 jours ou 28 jours, ont été faites selon la norme CEN 196-01.

La mesure des temps de début de prise a été réalisée selon la norme CEN 196-03.

### EXEMPLE 1 :

On prépare un mortier témoin (" MORTIER T ") selon la norme CEN 196-01, en mélangeant 450 g de ciment HP LAFARGE 52, 5 avec 1350 g de sable normalisé et 225 g d'eau.

On mesure l'étalement E en mm, le début de prise DP en heures et minutes ainsi que les résistances à 24 heures, 2 jours et 28 jours en MPa.

Ces résistances sont mesurées après stockage à 20°C.

On prépare de la même façon des compositions de mortier dans lesquelles on ajoute respectivement :
- MORTIER 1 : 0,3 % (en poids sec / poids sec de ciment) du PRODUIT B susmentionné, à savoir d'isosorbide cristallisé de haute pureté,
- MORTIER 2 : 1 % (sec/sec) de CaCl2,
- MORTIER 3 : 1 % (sec/sec) de Li2CO3
- MORTIER 4 : 1 % % (sec/sec) de NaOH,
- MORTIER 5 : 1 % % (sec/sec) de H2SO4, et
- MORTIER 6 : 2 % (sec/sec) du produit FRIOLITE®
commercialisé par SIKA comme adjuvant pour bétonnage par temps froid.

Les résultats obtenus pour le MORTIER T non adjuvanté et les mortiers 1 à 6 sont repris ci-après :

| MORTIER | E ( mm) | DP (h : min) | R24H (MPa) | R2J (MPa) | R28J (MPa) |
|---|---|---|---|---|---|
| MORTIER T | 232,5 | 5 : 00 | 10,1 | 24,4 | 48 |
| MORTIER 1 | 234,5 | 5 : 00 | 12,3 | 28 | 49 |
| MORTIER 2 | 242,5 | 2 : 50 | 17,3 | 34,4 | 54 |
| MORTIER 3 | 203,5 | 5 : 00 | 14,1 | 32,3 | 44,5 |
| MORTIER 4 | 228,5 | 4 : 15 | 15,8 | 30,1 | 40,5 |
| MORTIER 5 | 208 | 4 : 55 | 8,3 | 23,2 | 42,5 |
| MORTIER 6 | 206,5 | 4 : 30 | 12,9 | 25,3 | 44,5 |

Ces résultats montrent les avantages obtenus, dans le MORTIER 1, de par la mise en oeuvre d'un adjuvant selon l'invention constitué d'une composition à base d'isosorbide telle que le PRODUIT B.

Ce PRODUIT 3 n'a pas d'influence négative sur la plasticité ni sur le début de prise du mortier.

En regard des résultats obtenus avec le MORTIER T non adjuvanté, l'introduction du PRODUIT B permet d'améliorer significativement (de 21,3 %) la résistance au jeune âge du liant minéral.

Il en améliore également les résistances à 2 jours et 28 jours.

Les valeurs à 28 jours (R28J) sont même supérieures à celles obtenues avec des accélérateurs de prise et/ou de durcissement classiques comme Li2CO3 (MORTIER 3), NaOH (MORTIER 4), H2SO4(MORTIER 5) ou " FRIOLITE® " (MORTIER 6).

Certains de ces produits usuels (Li2CO3, H2S04 et " FRIOLITE® " ) ont par ailleurs un effet négatif sur la plasticité du mortier, la valeur d'étalement E obtenue (< 210 mm) étant largement inférieure à 95 % % de la valeur mesurée pour le témoin (232,5 mm).

Dans le cadre de cet EXEMPLE 1 il apparaît donc que c'est le PRODUIT B qui permet d'obtenir les performances les plus proches d'un adjuvant chloré constitué de CaCl2. Le PRODUIT 3 peut ici être considéré à la fois comme un agent accélérateur de durcissement et comme un agent améliorateur des propriétés mécaniques du mortier durci.

### EXEMPLE 2 :

On étudie la résistance des MORTIERS 1 à 6 précités, maintenus cette fois à basse température, à savoir à 5°C.

Les résultats obtenus sont repris ci-après

| | R24H (MPa) | R2J (MPa) | R28J (MPa) |
|---|---|---|---|
| MORTIER 1 | 2,3 | 12,3 | 62 |
| MORTIER 2 | 6,9 | 15 | 62,5 |
| MORTIER 3 | 1,8 | 12 | 49 |
| MORTIER 4 | 3,7 | 14,5 | 51 |
| MORTIER 5 | 1,8 | 5,9 | 48 |
| MORTIER 6 | 2,1 | 12,5 | 48,5 |

La valeur à 24 heures obtenue à 5°C pour le MORTIER 1 (2,3 MPa) est supérieure à 20 % de la valeur obtenue à 20°C pour le MORTIER T non adjuvanté (10,1 MPa). Ceci confirme le rôle accélérateur de durcissement joué par le PRODUIT B contenu dans le MORTIER 1.

Par ailleurs, les résultats de cet EXEMPLE 2 confirment le rôle améliorateur des propriétés mécaniques, joué par ce PRODUIT B.

De manière remarquable celui-ci permet, à 5°C, d'atteindre des performances à 28 jours pratiquement identiques à celles du CaCl2 et ce, malgré un taux d'introduction trois fois moindre.

### EXEMPLE 3 :

A partir d'un autre lot de ciment HP LAFARGE on réalise, comme décrit dans l'EXEMPLE 1, les compositions de mortier ci-après, adjuvantées ou non de 0,3 % (sec/sec) de l'un ou l'autre des dérivés de sucre suivants
- MORTIER T1 : sans adjuvant (témoin)
- MORTIER 7 : PRODUIT B
- MORTIER 8 : gluconate de sodium
- MORTIER 9 : sirop de glucose GLUCIDEX® 29*
- MORTIER 10 : dextrine TACKIDEX® DF 165*
- MORTIER 11 : : sirop de sorbitol NEOSORB® 70/02*
*commercialisé par la Demanderesse

On obtient les valeurs d'étalement et de début de prise ci-dessous.

| | E (mm) | DP (h : min) |
|---|---|---|
| MORTIER T1 | 235,5 | 3 : 30 |
| MORTIER 7 | 236 | 2 : 50 |
| MORTIER 8 | 265 | >15 : 00 |
| MORTIER 9 | 247 | 12 : 45 |
| MORTIER 10 | 225 | 6 : 30 |
| MORTIER 11 | 229 | 7 : 00 |

Ces résultats montrent globalement qu'un adjuvant selon l'invention comme le PRODUIT B se comporte différemment des autres adjuvants à base de dérivé de sucre, et notamment d'un sirop de sorbitol. Il permet d'accélérer le début de prise au mortier et ce, tout en lui maintenant une plasticité très convenable. La mesure, à 20°C , de la résistance à 17 heures a par ailleurs confirmé le rôle accélérateur de durcissement du PRODUIT B, celui-ci permettant d' atteindre une valeur de 10 MPa (MORTIER 7), significativement supérieure à celle obtenue pour le MORTIER T1 non adjuvanté (8,5 MPa).

### EXEMPLE 4 :

A partir d'un troisième lot de ciment H? LAFARGE on réalise, comme décrit dans l'EXEMPLE 1, les compositions de mortier ci-après, adjuvantées ou non de 0,3 % (sec/sec) de l'un ou l'autre des adjuvants selon l'invention suivants et qui ont été décrits ci-avant
- MORTIER T2 : sans adjuvant (témoin)
- MORTIER 12 : PRODUIT F
- MORTIER 13 : PRODUIT E
- MORTIER 14 : PRODUIT D
- MORTIER 15 : PRODUIT B

Pour l'ensemble de ces mortiers on obtient les valeurs ci-dessous d'étalement et de résistance à 24 heures, 3 jours et 28 jours, celles-ci étant mesurées après stockage à 20°C.

| | E (mm) | R24H (MPa) | R3J (MPa) | R28J (MPa) |
|---|---|---|---|---|
| MORTIER T2 | 222,5 | 13 | 30,8 | 45,5 |
| MORTIER 12 | 229 | 14,1 | 32,8 | 47 |
| MORTIER 13 | 228 | 14,2 | 31,9 | 46 |
| MORTIER 14 | 227,5 | 14,8 | 33,2 | 47,5 |
| MORTIER 15 | 225,5 | 15 | 35 | 47 |

Ces résultats confirment globalement l'intérêt de compositions à base de produit(s) de déshydratation interne d'un sucre hydrogéné comme le sorbitol, comme adjuvants de liants minéraux, en particulier comme agents accélérateurs de durcissement et comme agents améliorateurs des propriétés mécaniques des liants minéraux à l'état durci.

Ils montrent également que des produits comme les PRODUITS D et F, moins riches en isosorbide et sorbitan que les PRODUIT B ou D, peuvent très valablement être utilisés dans de telles applications.

### EXEMPLE 5 :

On étudie la résistance des MORTIERS 12 à 15 précités, maintenus cette fois à basse température, à savoir à 5°C.

Les résultats obtenus sont repris ci-après :

| | R24H (MPa) | R3J (MPa) | R28J (MPa) |
|---|---|---|---|
| MORTIER 12 | 3,6 | 17,4 | 58 |
| MORTIER 13 | 3,6 | 19,4 | 53 |
| MORTIER 14 | 3,7 | 20,0 | 52,5 |
| MORTIER 15 | 3,9 | 21,2 | 52 |

Pour l'ensemble de ces mortiers, la valeur obtenue à 24 heures à 5°C (3,6 à 3,9 MPa) représente largement plus de 20 % (en l'occurrence de 27,7 à 30 % environ) de la valeur obtenue à 24 heures, mais à 20°C, avec le MORTIER T2 non adjuvanté.

De même les valeurs obtenues à 28 jours à 5°C (52 à 58 MPa) représentent largement plus de 90 % (en l'occurrence de 114,3 à 127,5 %) de la valeur obtenue à 28 jours, mais à 20°C, avec le MORTIER T2 non adjuvanté.

Ceci confirme le rôle accélérateur de durcissement des PRODUITS B, D, E et F utilisables comme adjuvants selon l'invention.

Des tests supplémentaires ont montré que les PRODUITS A et C précités, bien que moins performants que les PRODUITS B, D, E, ou F, étaient également aptes, à basse température, à accélérer le durcissement et améliorer les caractéristiques du MORTIER T2.

### EXEMPLE 6 :

A partir d'un autre lot de ciment H? LAFARGE on réalise, comme décrit dans l'EXEMPLE **1,** les compositions de mortier ci-après, adjuvantées ou non de 0,4 % % (sec/sec) de l'un ou l'autre des adjuvants selon l'invention suivants :
- MORTIER T3 : sans adjuvant (témoin)
- MORTIER 16 : PRODUIT B
- MORTIER 17 : PRODUIT G
- MORTIER 18 : PRODUIT H

On obtient les valeurs d'étalement, de début de prise et de résistance à 17 heures (à 20°C) ci-dessous.

| | E (mm) | DP (h : min) | R17H (MPa) |
|---|---|---|---|
| MORTIER T3 | 219,5 | 3 : 20 | 10,8 |
| MORTIER 16 | 225 | 3 : 00 | 12,6 |
| MORTIER 17 | 214,5 | 3 : 05 | 12,5 |
| MORTIER 18 | 222,5 | 2 : 55 | 12,9 |

Ces résultats confirment que de tels adjuvants peuvent valablement être utilisés comme accélérateurs de prise et de durcissement.

Ils montrent, en regard des résultats obtenus précédemment, que, d'une manière générale, les adjuvants selon l'invention peuvent aussi bien être constitués de mélanges de plusieurs produits de déshydratation interne de sucres hydrogénés que contenir exclusivement ou très majoritairement un seul de ces produits (isosorbide, isomannide ou sorbitan, par exemple).

Des essais complémentaires menés sur plusieurs lots de ciment HTS LAFARGE 52,5 (faible teneur en C3A) ont confirmé par ailleurs la bonne aptitude de ces produits à être utilisés, à 20°C ou 5°C, en tant qu'agents accélérateurs de prise et/ou de durcissement ou agents améliorateurs des propriétés mécaniques de liants minéraux.

### EXEMPLE 7 :

A partir d'un autre lot de ciment HP LAFARGE on réalise, comme décrit dans l'Exemple 1, les compositions de mortier ci-après, adjuvantées ou non de l'un ou l'autre des adjuvants ci-après :
- MORTIER T4 :: Sans adjuvant (témoin),
- MORTIER 19 :: 0,3% de trioléate de sorbitan SPAN 85 tel que décrit dans le brevet JP 59 121143 susmentionné,
- MORTIER 20 :: 1,0% de sulfate de sodium,
- MORTIER 21 :: 1,0% de chaux,
- MORTIER 22 :: 0,3% de PRODUIT B,
- MORTIER 23 :: 0,3% de PRODUIT B + 1,0% de sulfate de sodium,
- MORTIER 24 :: 0,3% de PRODUIT B + 1,0% de chaux.

On obtient les valeurs d'étalement, de début de prise et de résistance à 24 heures (à 5 et 20°C) ci-dessous.

| MORTIER | E (mm) | DP (h : min) | R24H(5°C) (MPa) | R24H(20°C) (MPa) |
|---|---|---|---|---|
| T4 | 231 | 3 : 20 | 2,7 | 14,0 |
| 19 | 238 | 4 : 45 | 1,7 | 12,3 |
| 20 | 227 | 3 : 35 | 3,3 | 17,4 |
| 21 | 230 | 3 : 20 | 3,3 | 16,5 |
| 22 | 237 | 3 : 40 | 3,1 | 15,9 |
| 23 | 237 | 4 : 00 | 3,7 | 18,6 |
| 24 | 233 | 3 : 35 | 4,3 | 17,5 |

Ces résultats confirment l'intérêt du PRODUIT B, lequel se comporte différemment de l'adjuvant de type trioléate de sorbitan. En effet, ce dernier se comporte plutôt comme un produit retardateur de prise et de durcissement et ce, tant à 5°C qu'à 20°C. On note en outre qu'il peut être avantageux d'associer l'isosorbide (PRODUIT B) à des accélérateurs classiques tels que la chaux ou le sulfate de sodium.

Des essais complémentaires menés sur des ciments HTS LAFARGE 52,5 ont globalement confirmé l'ensemble de ces constatations.

## Revendications

1. Adjuvant pour liants minéraux **caractérisé en ce qu'**il comprend une composition contenant au moins un produit de déshydratation interne d'un sucre hydrogéné, de préférence d'un monosaccharide hydrogéné choisi parmi les hexitols, les pentitols, les tétritols et leurs mélanges, à l'exclusion du produit résultant de l'estérification par des chaînes grasses dudit produit de déshydratation interne d'un sucre hydrogéné.

2. Adjuvant selon la revendication 1 **caractérisé en ce que** ledit produit de déshydratation consiste en un hexitol déshydraté choisi parmi l'isosorbide, l'isomannide, le sorbitan, le mannitan et les mélanges quelconques d'au moins deux quelconques de ces produits.

3. Adjuvant selon l'une des revendications 1 ou 2 **caractérisé en ce que** ladite composition présente une teneur en produit(s) de déshydratation d'au moins 5 %, de préférence d'au moins 10 %, et plus préférentiellement encore d'au moins 40 %, ces pourcentages étant exprimés en poids sec total de produit(s) de déshydratation par rapport au poids sec de ladite composition.

4. Adjuvant selon la revendication 2 **caractérisé en ce que** ladite composition présente une teneur totale en isosorbide et sorbitan d'au moins 15 %, de préférence d'au moins 55 %, et plus préférentiellement encore d'au moins 70 %; ces pourcentages étant exprimés en poids sec total d'isosorbide et de sorbitan par rapport au poids sec de ladite composition.

5. Liant minéral **caractérisé en ce qu'**il contient de 0,001 à 5 % environ, de préférence de 0,01 à 2 %, d'un adjuvant selon l'une quelconque des revendications 1 à 4, ces pourcentages étant exprimés en poids sec dudit adjuvant par rapport au poids sec total de matière(s) première(s) pour ciment, de ciment et/ou de chaux contenu dans ledit liant minéral.

6. Utilisation d'un adjuvant selon l'une quelconque des revendications 1 à 4 pour la préparation d'un liant minéral.

7. Utilisation d'une composition contenant au moins un produit de déshydratation interne d'un sucre hydrogéné, de préférence d'un monosaccharide hydrogéné choisi parmi les hexitols, les pentitols, les tétritols et leurs mélanges, à l'exclusion du produit résultant de l'estérification par des chaînes grasses dudit produit de déshydratation interne d'un sucre hydrogéné, pour la préparation d'un adjuvant pour liant minéral ou d'un liant minéral.

8. Utilisation d'une composition selon la revendication 7, **caractérisée en ce que** le produit de déshydratation consiste en un hexitol déshydraté, de préférence choisi parmi l'isosorbide, l'isommanide, le sorbitan, le mannitan et les mélanges quelconques d'au moins deux quelconques de ces produits.

9. Utilisation d'un adjuvant selon la revendication 6 ou d'une composition selon l'une des revendications 7 t 8 comme agent accélérateur de prise et/ou de durcissement du liant minéral.

10. Utilisation d'un adjuvant selon la revendication 6 ou d'une composition selon l'une des revendications 7 et 8 comme agent améliorateur des propriétés mécaniques du liant minéral à l'état durci.

## Patentansprüche

1. Zusatz für mineralische Bindemittel, **dadurch gekennzeichnet, dass** er eine Zusammensetzung umfasst, die mindestens ein intemes Dehydratisierungsprodukt eines hydrierten Zuckers enthält, bevorzugt eines hydrierten Monosaccharids, ausgewählt aus den Hexiten, den Pentiten, den Tetriten und deren Gemischen, mit Ausnahme des Produkts, welches aus der Veresterung des internen Dehydratisierungsprodukt eines hydrierten Zuckers mit Fettketten entsteht.

2. Zusatz nach Anspruch 1, **dadurch gekennzeichnet, dass** das Dehydratisierungsprodukt aus einem dehydratisierten Hexit, ausgewählt aus Isosorbid, Isomannid, Sorbitan, Mannitan, und Gemischen von mindestens zwei dieser Produkte, besteht.

3. Zusatz nach einem der Ansprüche 1 oder 2, **dadurch gekennzeichnet, dass** die Zusammensetzung einen Gehalt an dem bzw. den Dehydratisierungsprodukten von mindestens 5 %, bevorzugt von mindestens 10 %, und noch stärker bevorzugt von mindestens 40 % aufweist, wobei diese Prozentanteile als Gesamttrockengewicht des bzw. der Dehydratisierungsprodukte bezogen auf das Trockengewicht der Zusammensetzung angegeben sind.

4. Zusatz nach Anspruch 2, **dadurch gekennzeichnet, dass** die Zusammensetzung einen Gesamtgehalt an Isosorbid und Sorbitan von mindestens 15 %, bevorzugt von mindestens 55 %, und noch stärker bevorzugt von mindestens 70 % aufweist, wobei diese Prozentanteile als Gesamttrockengewicht von Isosorbid und Sorbitan bezogen auf das Trockengewicht der Zusammensetzung angegeben sind.

5. Mineralisches Bindemittel, **dadurch gekennzeichnet, dass** es von 0,001 bis etwa 5 %, bevorzugt von 0,01 bis 2 % eines Zusatzes nach einem der Ansprüche 1 bis 4 enthält, wobei diese Prozentanteile als Trockengewicht des Zusatzes bezogen auf das Gesamttrockengewicht von dem bzw. den Zementausgangsmaterialien, Zement und/oder Kalk, die in dem mineralischen Bindemittel vorhanden sind, angegeben sind.

6. Verwendung eines Zusatzes nach einem der Ansprüche 1 bis 4 zur Herstellung eines mineralischen Bindemittels.

7. Verwendung einer Zusammensetzung, die mindestens ein internes Dehydratisierungsprodukt eines hydrierten Zuckers enthält, bevorzugt eines hydrierten Monosaccharids, ausgewählt aus den Hexiten, den Pentiten. den Tetriten und deren Gemischen, mit Ausnahme des Produkts, welches aus der Veresterung des internen Dehydratisierungsprodukt eines hydrierten Zuckers mit Fettketten entsteht, zur Herstellung eines Zusatzes für ein mineralisches Bindemittel oder eines mineralischen Bindemittels.

8. Verwendung einer Zusammensetzung nach Anspruch 7, **dadurch gekennzeichnet, dass** das Dehydratisierungsprodukt aus einem dehydratisierten Hexit, bevorzugt ausgewählt aus Isosorbid, Isomannid, Sorbitan, Mannitan, und Gemischen von mindestens zwei dieser Produkte, besteht.

9. Verwendung eines Zusatzes nach Anspruch 6 oder einer Zusammensetzung nach einem der Ansprüche 7 und 8 als Abbinde- und/oder Aushärtbeschleuniger des mineralischen Bindemittels.

10. Verwendung eines Zusatzes nach Anspruch 6 oder einer Zusammensetzung nach einem der Ansprüche 7 und 8 als Verbesserer der mechanischen Eigenschaften des mineralischen Bindemittels im ausgehärteten Zustand.

## Claims

1. Additive for mineral binders **characterized in that** it comprises a composition containing at least one product of internal dehydration, of a hydrogenated sugar, preferably of a hydrogenated monosaccharide selected from hexitols, pentitols, tetritols, and their mixtures, except the product resulting from the esterification by fatty chains of said internal dehydration product of a hydrogenated sugar.

2. Additive according to claim 1, **characterized in that** said dehydration product consists in a dehydrated hexitol selected from isosorbide, isomannide, sorbitan, mannitan and any mixtures of at least any two of these products.

3. Additive according to claim 1 or 2 **characterized in that** said composition has a content of dehydration product(s) of at least 5%, preferably at least 10%, and more preferably still of at least 40%, these percentages being expressed in total dry weight of product(s) of dehydration in relation to the dry weight of said composition.

4. Additive according to claim 2, **characterized in that** said composition has a total content of isosorbide and sorbitan of at least 15%, preferably at least 55%, and more preferably still of at least 70%, these percentages being expressed in total dry weight of isosorbide and of sorbitan in relation to the dry weight of said composition.

5. Mineral binder **characterized in that** it contains between about 0.001 and 5%, preferably between 0.01 and 2%, of an additive according to anyone of claims 1 to 4, these percentages being expressed in dry weight of said additive in relation to the total dry weight of raw material(s) for cement, of cement and/or of lime contained in said mineral binder.

6. Use of an additive according to anyone of claims 1 to 4, for the preparation of a mineral binder.

7. Use of a composition containing at least one product of internal dehydration of a hydrogenated sugar preferably a hydrogenated monosaccharide selected from hexitols, pentitols, tetritols, and their mixtures, except the product resulting from the esterification by fatty chains of said internal dehydration product of a hydrogenated sugar in an additive for a mineral binder or of a mineral binder.

8. Use of a composition according to claim 7, **characterized in that** the dehydration product consists of a dehydrated hexitol, preferably selected from isosorbide, isomannide, sorbitan, mannitan and any mixtures of at least any two of these products.

9. Use of an additive according to claim 6, or a composition according to claims 7 and 8 as an agent for accelerating setting and/or hardening of the mineral binder.

10. Use of an additive according to claim 6 or a composition according to claims 7 and 8 as an agent for improving the mechanical properties of the mineral binder in the hardened state.
